# EUROPEAN PATENT APPLICATION

(11) **EP 4 046 635 A1**
(43) Date of publication of application: **24.08.2022**
(21) Application number: 20876107.2
(22) Date of filing: 15.10.2020
(51) Int. Cl.: A61K 31/4178, A61K 31/437, A61K 31/553, A61K 31/713, A61P 17/14

(54) **COMPOSITION, COMPRISING RIP KINASE INHIBITOR, FOR PREVENTING HAIR LOSS OR PROMOTING HAIR REGROWTH**

(30) Priority: 17.10.2019 KR 20190129125; 19.02.2020 KR 20200020639; 12.08.2020 KR 20200101144
(71) Applicant: Epi Biotech Co., Ltd., Incheon 21984 (KR)
(72) Inventor: SUNG, Jong Hyuk, Seongnam-si Gyeonggi-do 13478 (KR); KIM, Young Soo, Incheon 21982 (KR)
(74) Representative: Callaghan, Dayle Anne
(86) International application number: PCT/KR2020/014074
(87) International publication number: WO 2021/075870

(57) **Abstract**

The present invention relates to a composition for preventing hair loss or promoting hair regrowth, the composition comprising a RIP kinase (RIPK) inhibitor. More specifically, the present invention relates to a composition for preventing hair loss or promoting hair regrowth, the composition comprising at least one RIP kinase inhibitor selected from the group consisting of a low molecular weight compound, siRNA, shRNA, or an antibody.

## Description

### Technical Field

The present invention relates to a composition for preventing hair loss or promoting hair regrowth, the composition comprising a RIP kinase (RIPK) inhibitor. More specifically, the present invention relates to a composition for preventing hair loss or promoting hair regrowth, the composition comprising at least one RIP kinase inhibitor selected from the group consisting of a low molecular weight compound, siRNA, shRNA, or an antibody.

### Background Art

Recently, as interest in beauty increases, interest in the treatment of alopecia is also increasing. Alopecia refers to a condition in which there is no hair where it should normally be. Although hair does not have an important physiological function directly related to life, it plays a very large role from a cosmetic point of view. In addition, hair has functions such as UV protection and head protection. If hair loss is severe, there can be problems in social life, and it can have a serious psychological impact. Thus, it is important in terms of quality of life.

Hair loss can be clinically divided into scarring alopecia accompanied by wounds and non-scarring alopecia in which only hair falls out. In the case of scarring alopecia, hair follicles are destroyed, so hair does not regrow. Hair is made in places referred to as hair follicles, and each hair follicle goes through a cycle of activity and resting phases. The time interval of such a hair cycle varies depending on the body part, but hair goes through a growth phase (anagen) of about 2 to 6 years and a regression phase (catagen) of 2 to 4 weeks, and then enters a resting phase (telogen) of 3 to 4 months. Each hair follicle has 10 to 20 hair follicle growth cycles in its lifetime.

Currently, the most commonly used surgical method for treating hair loss is autologous hair transplantation, and drug treatment using propecia and minoxidil is widely used. In the case of drug treatment, hair regrowth efficacy is exhibited at the time of administration, but hair loss proceeds again when treatment is stopped. In particular, in the case of minoxidil, there are side effects such as sexual dysfunction.

On the other hand, RIP kinase (receptor-interacting protein kinase, RIPK) is a TKL-family serine/threonine protein kinase related to innate immune signal transduction. In humans, there are five known RIP kinases such as RIPK1, RIPK2, RIPK3, RIPK4 and RIPK5. RIP kinase inhibitors are mainly studied for necrosis inhibition (Korean Patent Registration No. 10-1640068) or autoimmune treatment (Korean Patent Registration No. 10-1858346), but the contents in which these RIP kinase inhibitors are related to hair growth are not known.

In particular, necrostatin-1 and its derivative (necrostatin-1s, etc.), sibiriline, GSK963 or GSK2982772, which are known to block the RIP1/RIPK1 pathway, are tryptophan-based small molecule inhibitors of cell necrosis. In addition, they are known to specifically inhibit necroptosis, but it is not known that they have an effect of promoting hair growth.

Accordingly, in the present invention, as a result of efforts to develop a more effective composition for preventing and treating hair loss, it was confirmed that hair growth induction was promoted by at least one RIP kinase inhibitor selected from the group consisting of a low molecular weight compound, siRNA, shRNA, or an antibody, and the effect of hair regrowth in mice was significantly increased. Based on the above, the present inventors completed the present invention.

### Prior Art Document

### Patent Document

(Patent Document 1) Korean Patent Registration No. 10-1640068 (July 18, 2016)
(Patent Document 2) Korean Patent Registration No. 10-1858346 (May 16, 2018)

### Detailed Description of the Invention

### Technical Problem

An object of the present invention is to provide a composition for preventing hair loss or promoting hair regrowth, the composition comprising a RIP kinase inhibitor as an active ingredient.

### Solution to Problem

The present invention provides a composition for preventing hair loss or promoting hair regrowth, the composition comprising a RIP kinase inhibitor.

In addition, the present invention relates to a method for preventing hair loss or promoting hair regrowth using a RIP kinase inhibitor.

In one embodiment, the RIP kinase inhibitor may inhibit RIPK1 or RIPK3.

In one embodiment, the RIP kinase inhibitor may be a low molecular weight compound, siRNA, shRNA, or an antibody, but is not limited thereto.

In one embodiment of the present invention, the low molecular weight compound may be a compound of formula 1 below or a pharmaceutically acceptable salt thereof. 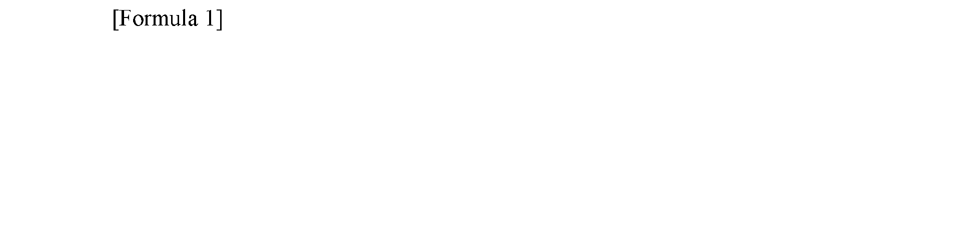 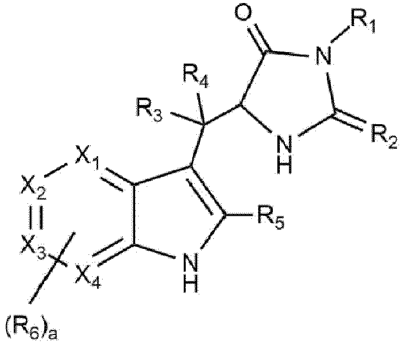 in which,
R₁ may be C₁-C₆ alkyl, C₂-C₆ alkenyl or C₂-C₆ alkynyl;
R₂ may be O or S;
R₃ to R₅ may be H, C₁-C₆ alkyl, C₂-C₆ alkenyl, C₂-C₆ alkynyl, aryl, heteroaryl, cycloalkyl or heterocycloalkyl;
R₆ may be each independently halo, alkoxy or hydroxy;
X₁ to X₄ may be each independently C or N; and
a may be an integer from 0 to 4.

In one embodiment, R₁ may be C₁-C₆ alkyl;
R₃ to R₅ may be H;
R₆ may be halo;
X₁ to X₄ may be C; and
a may be 0 or 1.

In another embodiment, the low molecular weight compound may be a compound of formula 2 or formula 3 below or a pharmaceutically acceptable salt thereof. The compound of formula 2 below represents necrostatin-1, and the compound of formula 3 below represents necrostatin-1s.

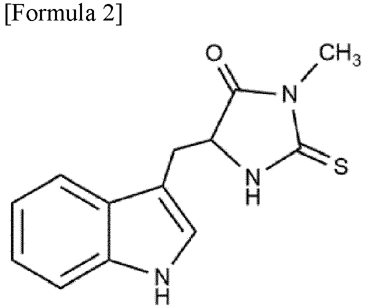

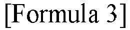

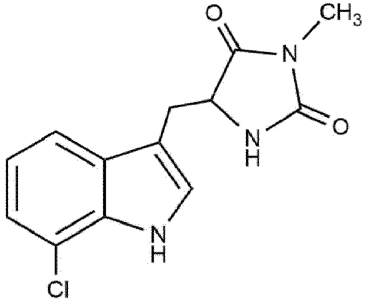

In one embodiment of the present invention, the low molecular weight compound may be a compound of formula 4 below or a pharmaceutically acceptable salt thereof. 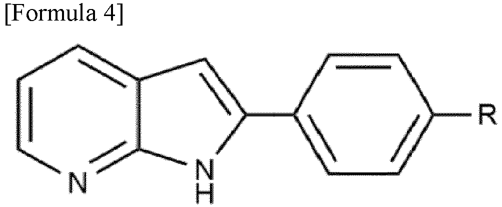 in which, R may be H, C₁-C₆ alkyl, C₂-C₆ alkenyl, C₂-C₆ alkynyl, halo, alkoxy, hydroxy, cyano, aryl, heteroaryl, cycloalkyl or heterocycloalkyl.

In another embodiment, the low molecular weight compound may be a compound of formula 5 below or a pharmaceutically acceptable salt thereof. The compound of formula 5 below is referred to as sibiriline.

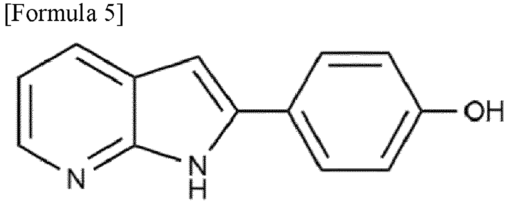

In one embodiment of the present invention, the low molecular weight compound may be a compound of formula 6 below or a pharmaceutically acceptable salt thereof. 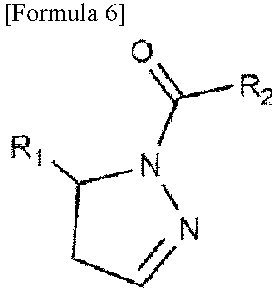 in which, R₁ or R₂ may be each independently H, C₁-C₆ alkyl, C₂-C₆ alkenyl, C₂-C₆ alkynyl, halo, alkoxy, hydroxy, cyano, aryl, heteroaryl, cycloalkyl or heterocycloalkyl.

In one embodiment, R₁ may be aryl, and R₂ may be C₁-C₆ alkyl.

In another embodiment, the low molecular weight compound may be a compound of formula 7 below or a pharmaceutically acceptable salt thereof. The compound of formula 7 below is referred to as GSK963.

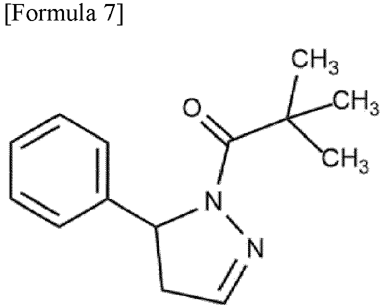

In one embodiment of the present invention, the low molecular weight compound may be a compound of formula 8 below or an isomer thereof or a pharmaceutically acceptable salt thereof. 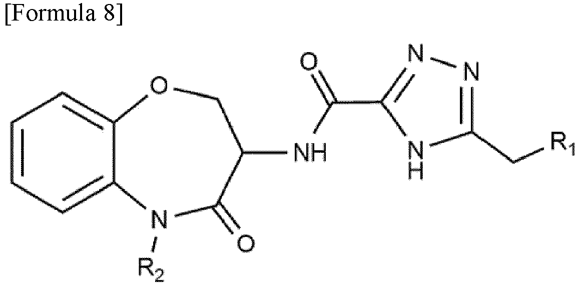 in which, R₁ or R₂ may be each independently H, C₁-C₆ alkyl, C₂-C₆ alkenyl, C₂-C₆ alkynyl, halo, alkoxy, hydroxy, cyano, aryl, heteroaryl, cycloalkyl or heterocycloalkyl.

In one embodiment, R₁ may be aryl, and R₂ may be C₁-C₆ alkyl.

In another embodiment, the low molecular weight compound may be a compound of formula 9 below or a pharmaceutically acceptable salt thereof. The compound of formula 9 below is referred to as GSK2982772.

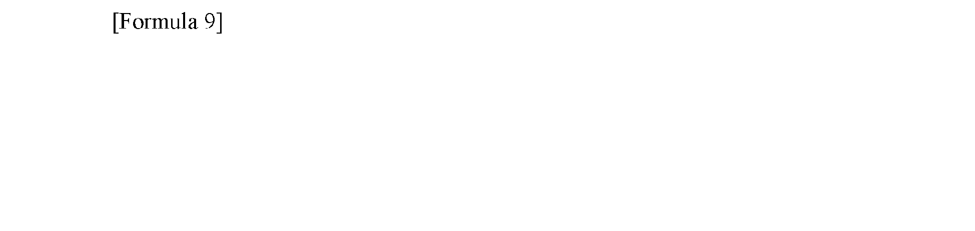

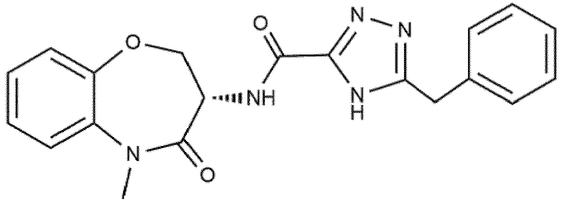

In one embodiment of the present invention, the siRNA may be siRNA comprising a pair of nucleotide sequences of SEQ ID NO: 1 and SEQ ID NO: 2, or siRNA comprising a pair of nucleotide sequences of SEQ ID NO: 3 and SEQ ID NO: 4.

In addition, the present invention provides a method for preventing or treating hair loss by administering a RIP kinase inhibitor to a patient having hair loss. In this case, the RIP kinase inhibitor may be the low molecular weight compound, siRNA, shRNA, or an antibody.

### Effects of the Invention

In the present invention, it was confirmed that the RIP kinase inhibitors necrostatin-1, necrostatin-1s, sibiriline, GSK963 or GSK2982772 significantly increase the effect of promoting hair growth induction in mouse vibrissae and the effect of hair regrowth in mice. In addition, it was confirmed that siRNA for a RIP kinase also promotes hair growth. Thus, the RIP kinase inhibitor of the present invention can be easily utilized for preventing hair loss or promoting hair regrowth.

### Brief Description of Drawings

Fig. 1 shows the degree of promoting hair growth induction when mouse vibrissae were treated with necrostatin-1 or necrostatin-1s.
Fig. 2 shows the results of (a) the appearance of hair growth and (b) the weight of the hair after necrostatin-1s was applied to a depilated mouse.
Fig. 3 shows the degree of promoting hair growth induction when mouse vibrissae were treated with sibiriline.
Fig. 4 shows the degree of promoting hair growth induction when mouse vibrissae were treated with GSK963.
Fig. 5 shows the degree of promoting hair growth induction when mouse vibrissae were treated with GSK2982772.
Fig. 6 shows the degree of promoting hair growth induction when mouse vibrissae were treated with the siRNA of the present invention.

### Best Mode for Carrying out the Invention

Hereinafter, embodiments and examples of the present invention will be described in detail with reference to the accompanying drawings so that those of ordinary skill in the art to which the present invention pertains can easily carry out. However, the present application may be implemented in several forms and is not limited to the embodiments and examples described herein.

Throughout the present specification, when a part "includes" a component, it means that other components may be further included, rather than excluding other components, unless otherwise stated.

The present invention relates to a composition for preventing hair loss or promoting hair regrowth, the composition comprising a RIP kinase inhibitor as an active ingredient. The RIP kinase inhibitor may be at least one selected from the group consisting of a low molecular weight compound, siRNA, shRNA, or an antibody, and may inhibit RIPK1 or RIPK3.

The low molecular weight compound is a compound having a low molecular weight, and includes a chemical substance capable of inhibiting the expression, transcription or activation of a RIP kinase-related gene.

Unless otherwise specified, the definitions of moieties and substituents used in the present invention have the following definitions and are used with the meanings commonly understood by those of ordinary skill in the art.

As used herein, "alkyl" is a hydrocarbon having primary, secondary, tertiary or quaternary carbon atoms and includes saturated aliphatic groups which may be straight-chain, branched or cyclic or a combination thereof. For example, an alkyl group may have from 1 to 20 carbon atoms (i.e., C₁-C₂₀ alkyl), from 1 to 10 carbon atoms (i.e., C₁-C₁₀ alkyl), or from 1 to 6 carbon atoms (i.e., C₁-C₆ alkyl). Examples of suitable alkyl groups include methyl (Me,-CH₃), ethyl (Et, -CH₂CH₃), 1-propyl (n-Pr, n-propyl, -CH₂CH₂CH₃), 2-propyl (i-Pr, i-propyl,-CH(CH₃)₂), 1-butyl (n-Bu, n-butyl, -CH₂CH₂CH₂CH₃), 2-methyl-1-propyl (i-Bu, i-butyl,-CH₂CH(CH₃)₂), 2-butyl (s-Bu, s-butyl, -CH(CH₃)CH₂CH₃), 2-methyl-2-propyl (t-Bu, t-butyl, -C(CH₃)₃), 1-pentyl (n-pentyl, -CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), and octyl (-(CH₂)₇CH₃), but are not limited thereto.

Moreover, as used throughout the specification, examples and claims, the term "alkyl" is intended to include both unsubstituted and substituted alkyl groups, the latter of which refers to an alkyl moiety having a substituent that replaces a hydrogen on at least one carbon of the hydrocarbon backbone, including haloalkyl groups such as trifluoromethyl and 2,2,2-trifluoroethyl, and the like.

The term "C_{x-y}" or "Cₓ-C_{y}" when used in conjunction with a chemical moiety such as alkyl, alkenyl or alkynyl is intended to include groups containing from x to y carbons in the chain. Coalkyl represents hydrogen when the group is in a terminal location or a bond when the group is in an internal location. For example, a C₁-C₂₀ alkyl group contains from 1 to 20 carbon atoms in the chain.

"Alkenyl" is a hydrocarbon that has primary, secondary, tertiary or quaternary carbon atoms, includes straight-chain, branched and cyclic groups or a combination thereof, and has at least one unsaturated region, i.e., carbon-carbon sp² double bond. For example, an alkenyl group may have from 2 to 20 carbon atoms (i.e., C₂-C₂₀ alkenyl), from 2 to 12 carbon atoms (i.e., C₂-C₁₂ alkenyl), from 2 to 10 carbon atoms (i.e., C₂-C₁₀ alkenyl), or from 2 to 6 carbon atoms (i.e., C₂-C₆ alkenyl). Examples of suitable alkenyl groups include vinyl (-CH=CH₂), allyl (-CH₂CH=CH₂), cyclopentenyl (-C₅H₇), and 5-hexenyl (-CH₂CH₂CH₂CH₂CH=CH₂), but are not limited thereto.

"Alkynyl" is a hydrocarbon that has primary, secondary, tertiary or quaternary carbon atoms, includes straight-chain, branched and cyclic groups or a combination thereof, and has at least one carbon-carbon sp triple bond. For example, an alkynyl group may have from 2 to 20 carbon atoms (i.e., C₂-C₂₀ alkynyl), from 2 to 12 carbon atoms (i.e., C₂-C₁₂ alkynyl), from 2 to 10 carbon atoms (i.e., C₂-C₁₀ alkynyl), or from 2 to 6 carbon atoms (i.e., C₂-C₆ alkynyl). Examples of suitable alkynyl groups include acetylenic (-C=CH) and propargyl (-CH₂C≡CH), but are not limited thereto.

As used herein, the term "halo" includes chloro, fluoro, bromo, and iodo.

"Cycloalkyl" refers to a non-aromatic, saturated or unsaturated, monovalent or divalent ring, which may be monocyclic, bicyclic or polycyclic, and wherein each atom of the ring is carbon. A cycloalkyl group may have from 3 to 7 carbon atoms as a monocycle, from 7 to 12 carbon atoms as a bicycle, and up to about 20 carbon atoms as a polycycle.

"Heterocycloalkyl" means that the ring structure of said cycloalkyl contains at least one heteroatom, preferably from 1 to 4 heteroatoms, more preferably from 1 to 2 heteroatoms. Examples of suitable heteroatoms may include oxygen, sulfur and nitrogen.

"Aryl" includes a monovalent or divalent aromatic hydrocarbon group, which is monocyclic, bicyclic or polycyclic, and wherein each atom of the ring is carbon. The aryl ring may have a 6- to 20-membered ring, and preferably 6- to 14-membered ring.

"Heteroaryl" refers to a monovalent or divalent aromatic group, which is monocyclic, bicyclic or polycyclic and contains at least one heteroatom in the ring. Examples of suitable heteroatoms may include oxygen, sulfur and nitrogen.

In the present invention, "siRNA" refers to a short double-stranded RNA capable of inducing an RNA interference (RNAi, RNA interference) phenomenon through cleavage of a specific mRNA. siRNA is composed of a sense RNA strand having the same sequence as the mRNA of a target gene and an antisense RNA strand having a complementary sequence thereto. Since siRNA can inhibit the expression of a target gene, it is provided by an efficient gene knock-down method or a gene therapy method.

siRNA is not limited to the complete pairing of double-stranded RNA parts that are paired with each other, and may include some parts that are not paired by mismatch (corresponding bases are not complementary), bulge (there are no corresponding bases in one chain), and the like. The entire length is 10 to 100 bases, preferably 15 to 80 bases, and more preferably 20 to 70 bases. The structure of the siRNA end can be either a blunt end or a cohesive end as long as it can inhibit the expression of a target gene by the RNAi effect. The structure of the cohesive end can be either a structure in which the 3' end protrudes or a structure in which the 5' end protrudes. The number of protruding bases is not limited. For example, the number of bases may be 1 to 8 bases, and preferably 2 to 6 bases.

In addition, the siRNA may include, for example, a low molecular weight RNA (for example, natural RNA molecules such as tRNA, rRNA, viral RNA or artificial RNA molecules) in the protruding part of one end within a range capable of maintaining the expression inhibitory effect of the target gene. The structure of the siRNA end does not need to have a cleaved structure on both sides, and may be a stem-loop structure in which one end portion of the double-stranded RNA is connected by a linker RNA. The length of the linker is not particularly limited as long as it does not interfere with forming a pair of stem portions.

The method for producing siRNA includes a method of directly synthesizing siRNA in vitro, then introducing it into a cell through a transfection process, and a method of transforming or transinfecting a cell with an siRNA expression vector or PCR-derived siRNA expression cassette prepared so that siRNA is expressed in a cell. The determination of how to prepare siRNA and introduce it into a cell or animal may vary depending on the purpose of the experiment and the cellular biological function of the target gene product.

In the present invention, "shRNA" refers to small hairpin RNA or short hairpin RNA (shRNA), and is RNA having a small hairpin structure used for silencing the expression of a gene through RNA interference. shRNA is introduced into a cell using a vector, and the shRNA hairpin structure is cleaved by other substances in the cell to become siRNA. This siRNAbinds to the RNA induced silencing complex (RISC), and this complex attaches to the mRNA having the sequence matching the siRNA and cleaves the mRNA. As a result, the mRNA is destroyed, so the gene of the mRNA is not expressed, resulting in gene silencing.

In the present invention, the siRNA is characterized in that it is an siRNA composed of a pair of nucleotide sequences of SEQ ID NO: 1 and SEQ ID NO: 2 or an siRNA composed of a pair of nucleotide sequences of SEQ ID NO: 3 and SEQ ID NO: 4, but may include a sequence that is 90% or more, 93% or more, 95% or more, 96% or more, 97% or more, 98% or more, or 99% or more identical to the nucleotide sequence of the siRNA.

The siRNA is characterized in that it preferably inhibits the expression of RIPK1 or RIPK3 gene, but is not limited thereto, and may inhibit the expression of other RIP kinase-related genes rather than RIPK1 or RIPK3.

The "antibody" is capable of inhibiting the activity of RIP kinase by specifically binding to RIP kinase (RIPK). The antibody may be used in a complete form having two full-length light chains and two full-length heavy chains, as well as in a fragment of the antibody molecule. A fragment of the antibody molecule refers to a fragment having at least a peptide tag (epitope) binding function, and includes single chain Fv (scFv), Fab, F (ab'), F (ab')₂, single domain, and the like.

The composition of the present invention may be prepared as a pharmaceutical composition, a quasi-drug composition or a cosmetic composition.

When the composition of the present invention is prepared as a pharmaceutical composition, the pharmaceutical composition of the present invention includes a pharmaceutically acceptable carrier. Pharmaceutically acceptable carriers included in the pharmaceutical composition of the present invention are commonly used, and include lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, and the like, but are not limited thereto. The pharmaceutical composition of the present invention may further include a lubricant, a wetting agent, a sweetening agent, a flavoring agent, an emulsifying agent, a suspending agent, a preservative, and the like, in addition to the above components.

The pharmaceutical composition of the present invention may be prepared in a unit dosage form by formulating using a pharmaceutically acceptable carrier and/or excipient according to a method that can be easily carried out by those of ordinary skill in the art to which the present invention pertains, or may be prepared by incorporation into a multi-dose container. In this case, the formulation may be in the form of a solution, suspension, syrup, or emulsion in oil or an aqueous medium, or may be in the form of an extract, pulvis, powder, granule, tablet or capsule, and may further include a dispersant or a stabilizer.

When the composition of the present invention is prepared as a quasi-drug composition, the RIP kinase inhibitor, which exhibits an effect of preventing hair loss or promoting hair regrowth, may be added as it is, or it may be used together with other quasi-drugs or quasi-drug ingredients, and may be appropriately used according to a conventional method.

The quasi-drug composition of the present invention for preventing hair loss or promoting hair regrowth is not particularly limited in its formulation, and it may be formulated in various forms in the form of quasi-drugs known in the art as exhibiting an effect of preventing hair loss or promoting hair regrowth. The formulated quasi-drugs include hair tonic, hair lotion, hair cream, hair spray, hair mousse, hair gel, hair conditioner, hair shampoo, hair rinse, hair pack, hair treatment, eyebrow hair regrowth agent, eyelash hair regrowth agent, eyelash nutrient agent, shampoo for pets, rinse for pets, hand sanitizer, detergent soap, soap, disinfectant, wet tissue, mask, ointment, patch or filter filler, and the like, and include all quasi-drugs in the ordinary sense.

In addition, in each formulation, the quasi-drug composition for preventing hair loss or promoting hair regrowth may be combined with other ingredients that are optionally selected according to the formulation or purpose of use of other quasi-drugs, etc.

When the composition of the present invention is prepared as a cosmetic composition, the components included in the cosmetic composition of the present invention include components commonly used in cosmetic compositions, in addition to the RIP kinase inhibitor as an active ingredient, for example, a conventional adjuvant such as an antioxidant, a stabilizer, a dissolving agent, vitamin, a pigment and a fragrance, and a carrier.

The cosmetic composition of the present invention may be prepared in any formulation conventionally prepared in the art. The cosmetic composition of the present invention may be formulated, for example, but not limited to, as a solution, suspension, emulsion, paste, gel, cream, lotion, powder, soap, surfactant-containing cleansing oil, powder foundation, emulsion foundation, wax foundation, spray, and the like.

Hereinafter, the present invention will be described in more detail through the following examples, but the following examples are for illustrative purposes only and are not intended to limit the scope of the present invention.

### [Example 1]

### Confirmation of effect of inducing hair growth according to necrostatin-1 or necrostatin-1s treatment

In order to confirm the effect of inducing hair growth according to the RIP kinase inhibitor necrostatin-1 (Nec-1, formula 2) or necrostatin-1s (Nec-1s, formula 3) treatment, the following inactive type necrostatin-1 (Nec-1i, formula 10) was used as a control group to conduct an experiment.

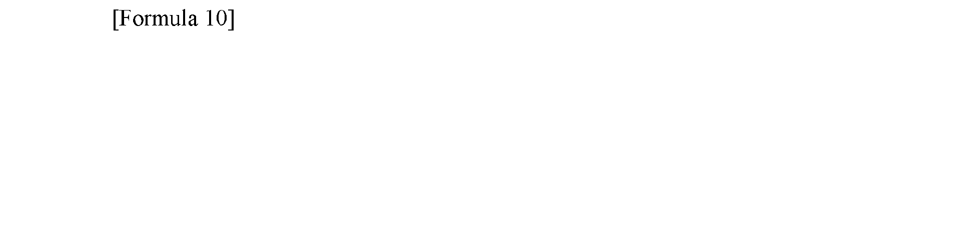

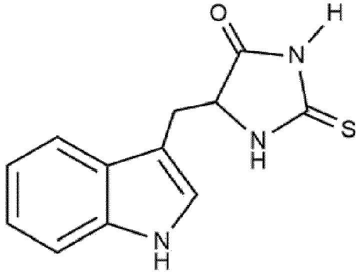

The mouse vibrissae were treated with the above inactive type necrostatin-1 and the necrostatin-1 and necrostatin-1s of the present invention to confirm the degree of hair growth induction.

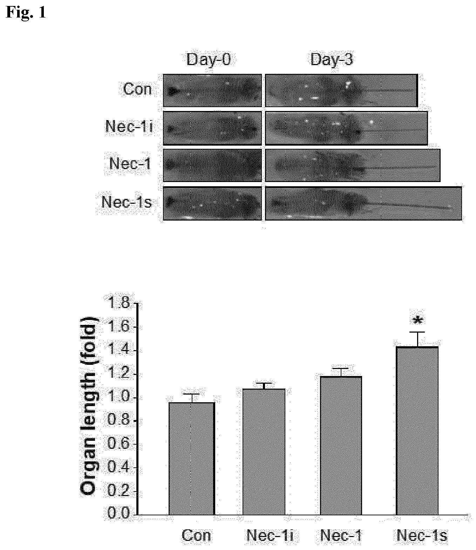

Specifically, the vibrissae of C57BL/6 female mice were trimmed and organ cultured. The trimmed vibrissae were cultured in a 48-well plate so as to become one per well, and divided into a total of 4 groups: the untreated group, the group treated with the inactive type necrostatin-1, the group treated with necrostatin-1, and the group treated with necrostatin-1s. The vibrissae of the three treatment groups were treated with 10 µM, respectively, and then cultured for 3 days in an incubator at 37 °C. The results are shown in Fig. 1.

As shown in Fig. 1, it was confirmed that necrostatin-1 and necrostatin-1s had an excellent effect of promoting hair growth compared to the inactive type necrostatin-1.

### [Example 2]

### Confirmation of hair regrowth effect according to necrostatin-1s treatment

The hairs of ten 6-week-old male C₃H/HeN mice were shaved short using a depilatory machine, and the remaining hairs were completely removed using a depilatory agent, and then they were stabilized for one day before use in the experiment.

Mice were divided into 5 mice per group under the following conditions to conduct an experiment.
(i) Untreated control group
(ii) Group administered with 2% minoxidil (MNX)
(iii) Group administered with 10 µM necrostatin-1s
(iv) Group administered with 100 µM necrostatin-1s

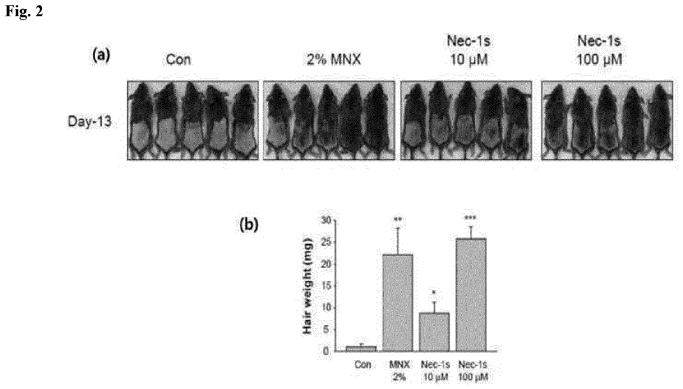

For each group, 100 µl was applied to the back of the mouse for a total of 13 times on the 13th day, and the degree of hair regrowth on the back of the mouse was observed for 13 days. After 13 days, the hairs on the back of the mouse in which hair regrowth occurred were shaved using a razor, and then the hairs were obtained, and the weight was measured. The results are shown in Fig. 2.

As shown in Fig. 2, it was confirmed that the group administered with necrostatin-1s exhibited an excellent hair growth effect compared to the untreated control group. In particular, it was confirmed that the group administered with 100 µM necrostatin-1s had a more excellent effect of inducing hair growth compared to the group administered with commercially available minoxidil.

### [Example 3]

### Confirmation of effect of inducing hair growth according to sibiriline treatment

In order to confirm the effect of inducing hair growth according to the RIP kinase inhibitor sibiriline (formula 5) treatment, the degree of hair growth induction was confirmed by treating the mouse vibrissae with sibiriline.

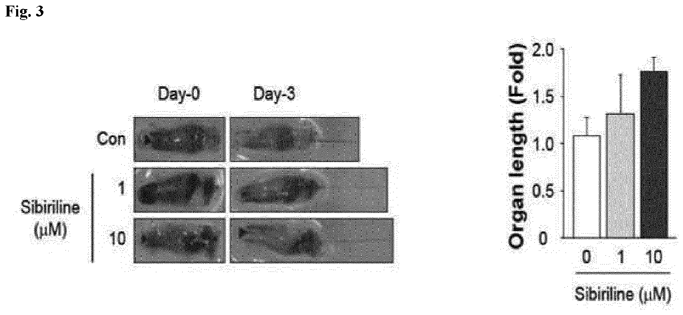

Specifically, the vibrissae of C57BL/6 female mice were trimmed and organ cultured. The trimmed vibrissae were cultured in a 48-well plate so as to become one per well, and divided into a total of 3 groups: the untreated group (control group), the group treated with 1 µM sibiriline, and the group treated with 10 µM sibiriline. The vibrissae were treated, respectively, and then cultured for 3 days in an incubator at 37 °C. The results are shown in Fig. 3.

As shown in Fig. 3, it was confirmed that the groups treated with 1 µM and 10 µM sibiriline had an excellent efficacy of promoting hair growth.

### [Example 4]

### Confirmation of effect of inducing hair growth according to GSK963 treatment

In order to confirm the effect of inducing hair growth according to the RIP kinase inhibitor GSK963 (formula 7) treatment, the degree of hair growth induction was confirmed by treating the mouse vibrissae with GSK963.

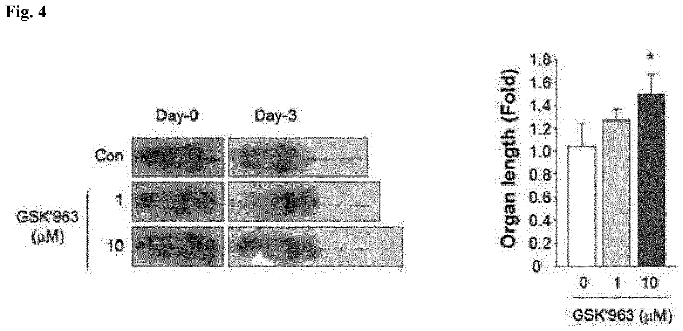

Specifically, the vibrissae of C57BL/6 female mice were trimmed and organ cultured. The trimmed vibrissae were cultured in a 48-well plate so as to become one per well, and divided into a total of 3 groups: the untreated group (control group), the group treated with 1 µM GSK963, and the group treated with 10 µM GSK963. The vibrissae were treated, respectively, and then cultured for 3 days in an incubator at 37 °C. The results are shown in Fig. 4.

As shown in Fig. 4, it was confirmed that the groups treated with 1 µM and 10 µM GSK963 had an excellent efficacy of promoting hair growth.

### [Example 5]

### Confirmation of effect of inducing hair growth according to GSK2982772 treatment

In order to confirm the effect of inducing hair growth according to the RIP kinase inhibitor GSK2982772 (formula 9) treatment, the degree of hair growth induction was confirmed by treating the mouse vibrissae with GSK2982772.

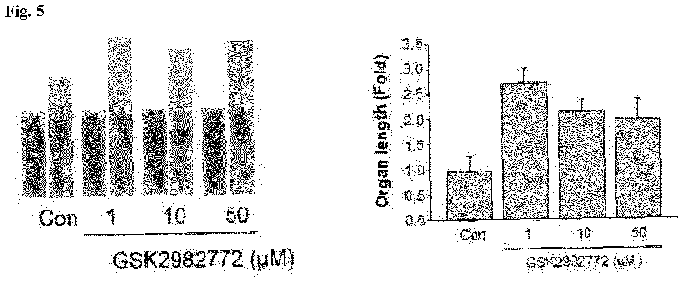

Specifically, the vibrissae of C57/BL6 female mice were trimmed and organ cultured. The trimmed vibrissae were cultured in a 48-well plate so as to become one per well, and divided into a total of 4 groups: the untreated group (control group), the group treated with 1 µM GSK2982772, the group treated with 10 µM GSK2982772, and the group treated with 50 µM GSK2982772. The vibrissae were treated, respectively, and then cultured for 3 days in an incubator at 37 °C. The results are shown in Fig. 5.

As shown in Fig. 5, it was confirmed that all of the groups treated with 1 µM, 10 µM, and 50 µM GSK2982772 had an excellent efficacy of promoting hair growth.

### [Example 6]

### Confirmation of effect of inducing hair growth according to siRNA treatment on RIP kinase

In order to confirm the effect of inducing hair growth according to siRNA treatment capable of inhibiting RIP kinase expression, siRNA was synthesized as shown in Table 1 below, and control siRNA as a control group was purchased from BIONEER CORPORATION and used.

**[Table 1]**

| RIP kinase expression inhibition siRNA sequence | | |
|---|---|---|
| Name | Nucleotide sequence (5'->3') | SEQ ID NO: |
| RIPK1 siRNA siRNA No (19766-1) | GUCCUUGUGUUACCACAGA | 1 |
| | UCUGUGGUAACACAAGGAC | 2 |
| RIPK3 siRNA siRNA No (56532-1) | CGAGUGAUGUCUACAGCUU | 3 |
| | AAGCUGUAGACAUCACUCG | 4 |

The degree of hair growth induction was confirmed by treating the mouse vibrissae with the siRNA of Table 1 above.

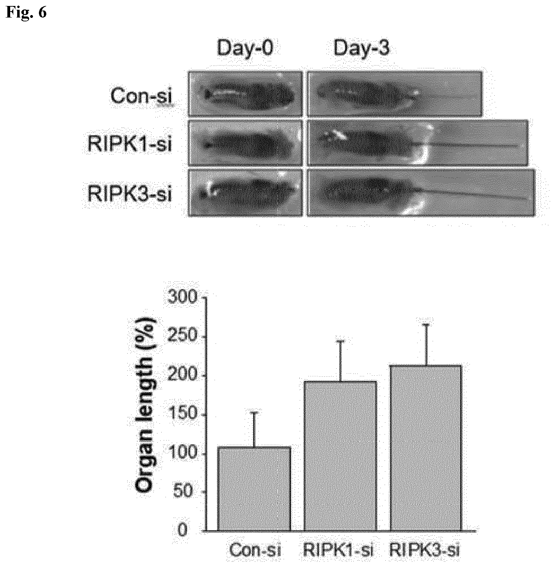

Specifically, the vibrissae of C57BL/6 female mice were trimmed and organ cultured. The trimmed vibrissae were cultured in a 48-well plate so as to become one per well, and divided into a total of 3 groups: the group treated with RIPK1 siRNA, the group treated with RIPK3 siRNA, and the group treated with control siRNA (control group) in Table 1. The vibrissae were treated, respectively, and then cultured for 3 days in an incubator at 37 °C. The results are shown in Fig. 6.

As shown in Fig. 6, it was confirmed that hair growth was promoted by treatment with RIPK1 siRNA and RIPK3 siRNA.

From the above experimental results, it can be seen that hair growth is promoted by inhibition of RIP kinase expression. Thus, it can be seen that other RIP kinase inhibitors rather than the siRNA used in the present invention also have an effect of preventing hair loss or promoting hair regrowth.

## Claims

1. A composition for preventing hair loss or promoting hair regrowth, the composition comprising a RIP kinase (receptor-interacting protein kinase) inhibitor.

2. The composition for preventing hair loss or promoting hair regrowth according to claim 1, **characterized in that** the RIP kinase inhibitor is at least one selected from the group consisting of a low molecular weight compound, siRNA, shRNA, or an antibody.

3. The composition for preventing hair loss or promoting hair regrowth according to claim 2, **characterized in that** the low molecular weight compound is a compound of formula 1 below or a pharmaceutically acceptable salt thereof: 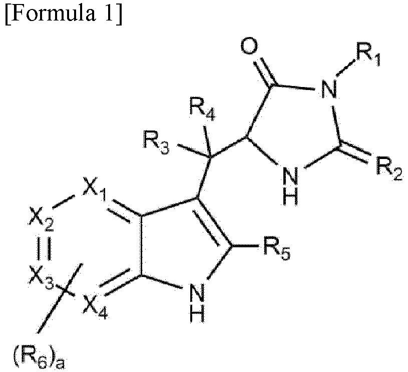
in which,
R₁ is C₁-C₆ alkyl, C₂-C₆ alkenyl or C₂-C₆ alkynyl;
R₂ is O or S;
R₃ to R₅ are H, C₁-C₆ alkyl, C₂-C₆ alkenyl, C₂-C₆ alkynyl, aryl, heteroaryl, cycloalkyl or heterocycloalkyl;
R₆ is each independently halo, alkoxy or hydroxy;
X₁ to X₄ are each independently C or N; and
a is an integer from 0 to 4.

4. The composition for preventing hair loss or promoting hair regrowth according to claim 3, **characterized in that**
R₁ is C₁-C₆ alkyl;
R₃ to R₅ are H;
R₆ is halo;
X₁ to X₄ are C; and
a is 0 or 1.

5. The composition for preventing hair loss or promoting hair regrowth according to claim 3, **characterized in that** the low molecular weight compound is a compound of formula 2 or formula 3 below or a pharmaceutically acceptable salt thereof:

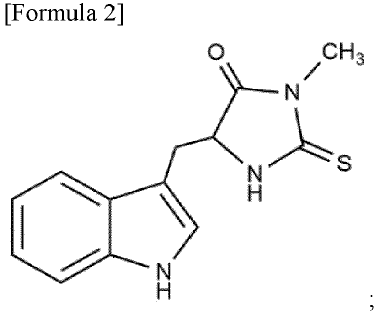

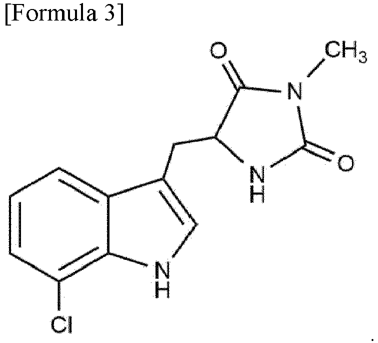

6. The composition for preventing hair loss or promoting hair regrowth according to claim 2, **characterized in that** the low molecular weight compound is a compound of formula 4 below or a pharmaceutically acceptable salt thereof: 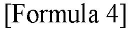 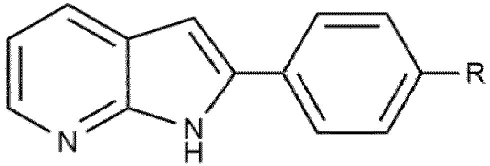 in which, R is H, C₁-C₆ alkyl, C₂-C₆ alkenyl, C₂-C₆ alkynyl, halo, alkoxy, hydroxy, cyano, aryl, heteroaryl, cycloalkyl or heterocycloalkyl.

7. The composition for preventing hair loss or promoting hair regrowth according to claim 6, **characterized in that** the low molecular weight compound is a compound of formula 5 below or a pharmaceutically acceptable salt thereof:

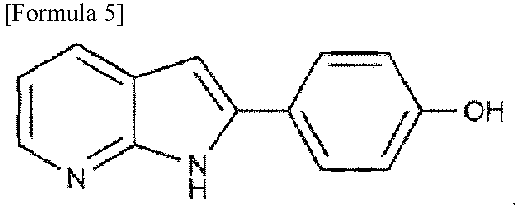

8. The composition for preventing hair loss or promoting hair regrowth according to claim 2, **characterized in that** the low molecular weight compound is a compound of formula 6 below or a pharmaceutically acceptable salt thereof: 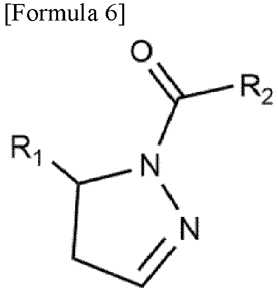 in which, R₁ or R₂ is each independently H, C₁-C₆ alkyl, C₂-C₆ alkenyl, C₂-C₆ alkynyl, halo, alkoxy, hydroxy, cyano, aryl, heteroaryl, cycloalkyl or heterocycloalkyl.

9. The composition for preventing hair loss or promoting hair regrowth according to claim 8, **characterized in that**
R₁ is aryl; and
R₂ is C₁-C₆ alkyl.

10. The composition for preventing hair loss or promoting hair regrowth according to claim 8, **characterized in that** the low molecular weight compound is a compound of formula 7 below or a pharmaceutically acceptable salt thereof:

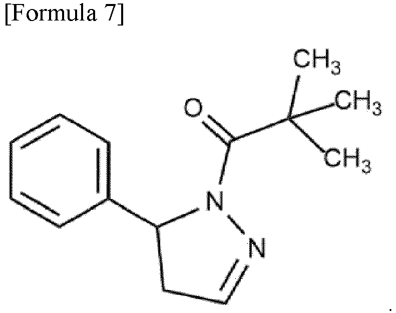

11. The composition for preventing hair loss or promoting hair regrowth according to claim 2, **characterized in that** the low molecular weight compound is a compound of formula 8 below or an isomer thereof or a pharmaceutically acceptable salt thereof: 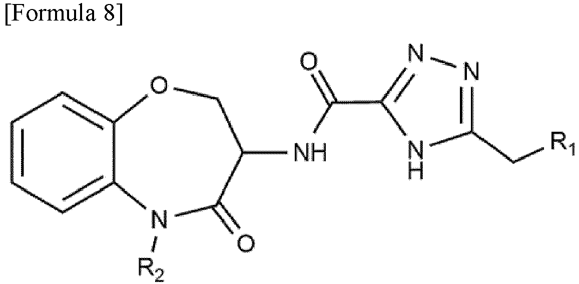 in which, R₁ or R₂ is each independently H, C₁-C₆ alkyl, C₂-C₆ alkenyl, C₂-C₆ alkynyl, halo, alkoxy, hydroxy, cyano, aryl, heteroaryl, cycloalkyl or heterocycloalkyl.

12. The composition for preventing hair loss or promoting hair regrowth according to claim 11, **characterized in that**
R₁ is aryl; and
R₂ is C₁-C₆ alkyl.

13. The composition for preventing hair loss or promoting hair regrowth according to claim 11, **characterized in that** the low molecular weight compound is a compound of formula 9 below or a pharmaceutically acceptable salt thereof:

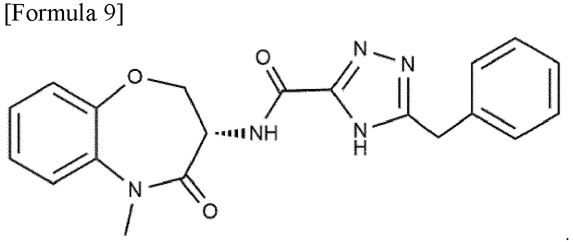

14. The composition for preventing hair loss or promoting hair regrowth according to claim 2, **characterized in that** the siRNA is siRNA comprising a pair of nucleotide sequences of SEQ ID NO: 1 and SEQ ID NO: 2, or siRNA comprising a pair of nucleotide sequences of SEQ ID NO: 3 and SEQ ID NO: 4.
